# EUROPEAN PATENT APPLICATION

(11) **EP 0 577 026 A2**
(43) Date of publication of application: **05.01.1994**
(21) Application number: 93110208.1
(22) Date of filing: 25.06.1993
(51) Int. Cl.: B01D 53/32, B01D 46/24, B01D 35/06

(54) **Filtration method and filter device**

(30) Priority: 29.06.1992 JP 231244/92; 14.01.1993 JP 44274/93
(71) Applicant: Nagaura, Yoshiaki, Chikushino-shi Fukuoka-ken (JP)
(72) Inventor: Nagaura, Yoshiaki, Chikushino-shi Fukuoka-ken (JP)
(74) Representative: Goddar, Heinz J., Dr.

(57) **Abstract**

A filter device having a plurality of vapor-permeable conductive electrodes laminated at intervals or having an insulating filter sandwiched between the vapor-permeable electrodes is disposed in an insulating pipe, and various vapors or liquids, such as exhaust gas, city tap water, sewage, seawater or blood, are passed through the device for filtration. Using the device, carbon dioxide, sulfur compounds, nitrogen compounds and others may be well decomposed and the soot from them may be well recovered; and microorganisms such as bacteria, viruses and others in city tap water, sewage, seawater, blood and other solutions may well be inactivated or killed.

## Description

### FIELD OF THE INVENTION

The present invention relates to a filtration method and a filter device which uses a laminate electrode especially (a) for decomposing carbon dioxide, sulfur compounds, or nitrogen compounds discharged from a flue or exhaust gas duct, and for recovering the soot from these compounds; (b) for removing compounds such as ammonia and bilirubin, as well as microorganisms such as bacteria and viruses that may exist in city tap water, sewage, sea water, blood, and other solutions; and (c) for removing microorganisms such as bacteria and viruses that may exist in air, gas, or soil.

### BACKGROUND OF THE INVENTION

Heretofore, zeolite and ammonia have been used as a catalysts for decomposing sulfur compounds and nitrogen compounds.

It has been said that selective reduction of nitrogen monoxide (NO) discharged from a diesel engine having a highly dissolved oxygen concentration or from a gasoline engine of a dilution combustion system under an oxidizing atmosphere is possible only with ammonia, but it is pointed out that ammonia is expensive and dangerous. W. Held et al. reported on the basis of their discovery of a Cu-ZSM-5 catalyst that various catalysts are active to the reaction of a process of removing nitrogen monoxide (W. Weld, A. Konig, T, Richter, L. Puppe; SAE Paper 900496 (Feb., 1990)). Iwamoto et al. reported that a copper ion-exchanging ZSM-5 zeolite (Cu-MFI) provides a high activity in the catalytic decomposition of NO which has heretofore been said to be difficult (M. Iwamoto, H. Yahiro, K. Tanada, N. Mizuno, Y. Mine, S. Kagawa; J. Phys. Chem., 95, 3727 (1991)).

However, the method of decomposing nitrogen monoxide with a catalyst of such conventional zeolite and others required a high temperature of from 200 to 500°C and therefore involved a problem that the catalyst could not be used at room temperature.

In order to inactivate bacteria and viruses as they exist in the blood, vaccines or chemicals have heretofore been used. However, where bacteria or viruses of novel species appear, studies and experiments for obtaining suitably effective vaccines or chemicals require a great deal of time and money. At present, there are some known viruses to which no effective vaccine has yet been found.

### SUMMARY OF THE INVENTION

One object of the present invention is to electrically decompose carbon dioxide, sulfur compounds, nitrogen compounds and others, using an electrode carbon, electric voltage and electric current as catalysts, and to recover the soot and other materials from the decomposates.

Another object of the present invention is to use electric voltage and electric current to kill microorganisms such as bacteria, viruses and the like as they exist in city tap water, sewage, seawater, blood and other solutions and vapors.

Specifically, in accordance with the present invention, a filter device having a plurality of vapor-permeable conductive electrodes laminated at intervals or having an insulating filter sandwiched between the vapor-permeable electrodes, is disposed in an insulating pipe, and various vapors or liquids are passed through the device for filtration. Since the electrode structure of the device is such that the distance between the respective electrodes may be extremely small, the potential gradient may be enlarged so that the electric voltage and current as applied to the device may be utilized more efficiently. By using the device, carbon dioxide, sulfur compounds, nitrogen compounds and others may well be decomposed and the resulting soot can effectively be recovered. Additionally, bacteria, viruses and other microorganisms that are in city tap water, sewage, seawater, blood and other solutions can be efficiently inactivated or killed.

### BRIEF EXPLANATION OF THE DRAWINGS

Fig. 1 to Fig. 8 are each longitudinal sectional views of an embodiment of the present invention.

Fig. 9 is a plan view of a vapor-permeable electrode having terminals at both ends in which the electrode is used inside a filter tool.

Fig. 10 to Fig. 15 are each longitudinal sectional views of an embodiment of the present invention.

Fig. 16 is an enlarged view of the electrode part of the embodiment of Fig. 15.

Fig. 17 is a plan view of a vapor-permeable electrode.

Fig. 18 is a plan view of a filter.

Fig. 19 and Fig. 20 are each longitudinal sectional views of an embodiment of the present invention.

Fig. 21 is an enlarged view of the electrode of the embodiment of Fig. 20.

Fig. 22 is a plan view of a vapor-permeable electrode.

Fig. 23 is a plan view of a filter.

Fig. 24 to Fig. 26 are each longitudinal sectional views of an embodiment of the present invention.

Fig. 27 and Fig. 28 are each enlarged views of an electrode.

Fig. 29 is a plan view of a vapor-permeable electrode.

Fig. 30 is a plan view of a filter.

Fig. 31 and Fig. 32 are each longitudinal sectional views of an embodiment of the present invention.

Fig. 33 and Fig. 34 are each enlarged views of an electrode.

Fig. 35 is a plan view of a vapor-permeable electrode.

Fig. 36 is a lateral sectional view of an embodiment of the present invention.

Fig. 37 is a side view of an embodiment of the present invention.

Fig. 38 is a lateral sectional view of an embodiment of the present invention.

Fig. 39 is a side view of an embodiment of the present invention.

Fig. 40 to Fig. 42 are each longitudinal sectional views of an embodiment of the present invention.

Fig. 43 is a lateral sectional view of an embodiment of the present invention.

Fig. 44 is a longitudinal sectional view of showing the embodiment of the present invention.

### DESCRIPTION OF PREFERRED EMBODIMENTS

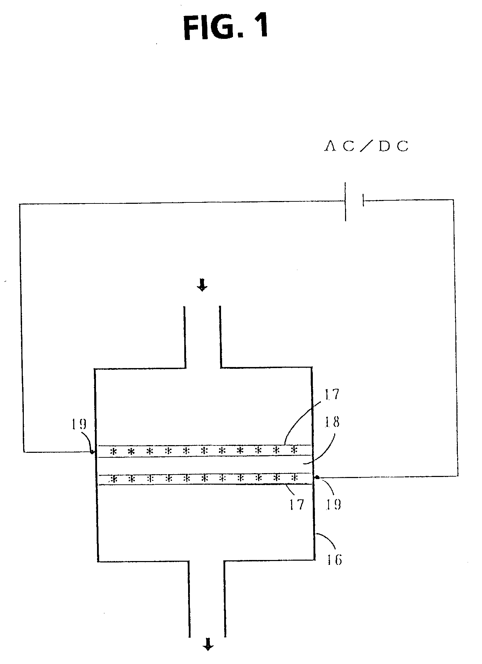

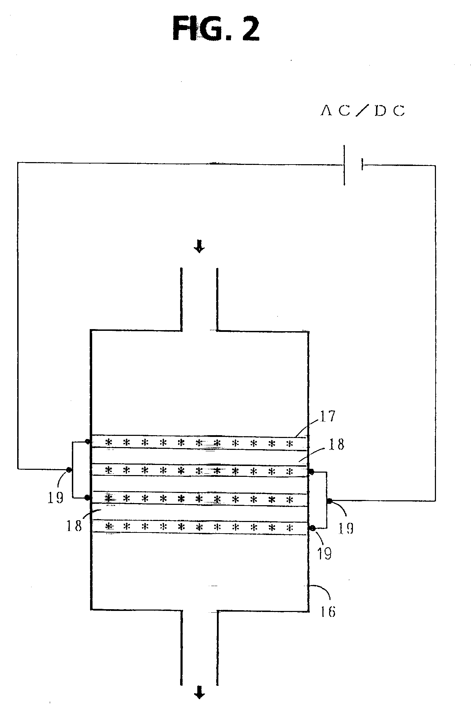

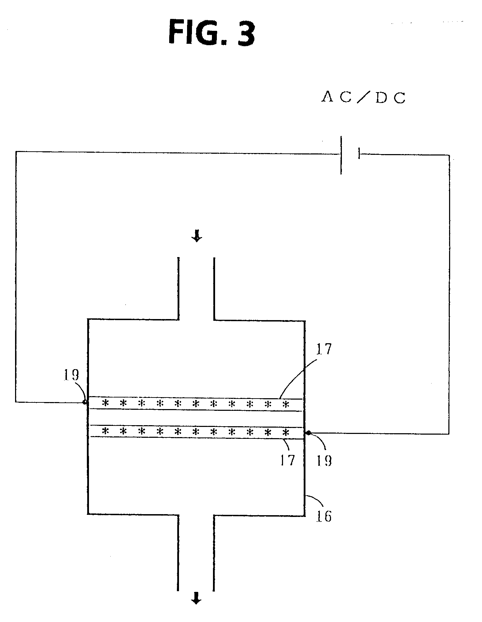

One embodiment of the present invention is shown in Fig. 1, in which a filter 18 made of a vapor-permeable insulating material, such as filter paper or a sieve, is sandwiched between a vapor-permeable electrode 17 made of carbon fibers, graphite, gold, platinum, ferrum, titanium, or carbon and another vapor-permeable electrode 17 made of the same material so that the two vapor-permeable electrodes 17 and the vapor-permeable insulating filter 18 are laminated, and where the filter 18 is used as filter paper or a sieve. Where an aqueous solution, seawater, blood or other solutions are passed through the inside of the vapor-permeable electrodes 17, the potential frequency may be kept high even though the potential difference is small, since the distance between the electrode 17 and the electrode 17 is small.

Since the distance between the two electrodes 17 may be adjusted by changing the thickness of the vapor-permeable insulating filter 18 such as filter paper or a sieve, the distance between the electrodes 17 and 17 can be made smaller to increase the potential gradient, even under the condition of a small potential difference. In addition to this, the embodiment has the following advantages:
(1) Since the electric current applied to the device passes only the distance between the two electrodes 17 and 17, it therefore passes only while the solution to be applied to the device passes through it, and the current may be passed through the solution even though the solution is an electrolytic solution such as seawater or blood, hardly causing any electrolysis of the solution.
(2) By making the distance between the two electrodes 17 extremely small, an alternating current such as a bi-polar pulse, sine wave alternating current or the like may be applied to the device, whereupon even though the electrodes 17 change alternately from positive to negative, positively or negatively charged microorganisms such as bacteria, viruses and others may be moved forward and backward between the electrodes 17 and 17 so as to be trapped in the space between the electrodes 17 and 17, and therefore the microorganisms are seemingly absorbed by the electrodes.
(3) Even though any power source of a direct current or an alternating current may be used, positively or negatively charged microorganisms such as bacteria, viruses and others cannot pass through the vapor-permeable electrodes 17.
(4) Since a charged sieve may be made of carbon fibers, the sieve may have a mesh size suitable to the size of the grains to be applied thereto.

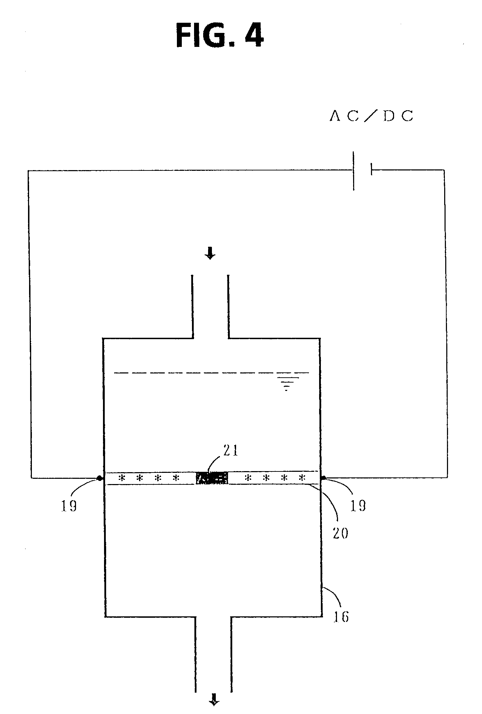

As another embodiment, the present invention may employ a vapor-permeable electrode 20, which is composed of a filter made of carbon fibers, graphite, gold, platinum, ferrum, titanium, or carbon and terminals 19 attached to both ends of the filter, such as a filter paper or a sieve, as shown in Fig. 4. Where an aqueous solution, seawater, blood and other solutions are passed through the vapor-permeable electrode 20, the current applied thereto passes through the easy site to yield the following advantages:
(1) Since almost all the current passes through the upper site of the vapor-permeable electrode 20, it hardly causes any electrolysis of the solutions.
(2) Even though any power source of a direct current or an alternating current may be used, positively or negatively charged microorganisms such as bacteria, viruses and others cannot pass through the vapor-permeable electrode 20. This is because when negatively charged viruses go to the negatively charged site they would be repelled by it, but when they go to the positively charged site they would be absorbed by it. Thus, the charged microorganisms cannot pass through the electrode 20. However, since only the center part of the vapor-permeable electrode 20 has a potential difference of 0 (zero), it is recommended that only the center part of the electrode 20 is so modified that the solution applied thereto cannot permeate therethrough.
(3) Even though a power source such as bi-polar pulse, sine wave alternating current is used, the electrode 20 can always absorb microorganisms such as bacteria, viruses and others so that the electrolysis of the solution applied to the device can be suppressed to a minimum degree.
(4) The solution applied to the device is influenced by the current only when it passes through the vapor-permeable electrode 20.
(5) Using the electrode 20 of this type, an electric filter paper or sieve may be formed, which may selectively separate charged microorganisms such as bacteria, viruses and others by the use of an electric current.

Fig. 1 to Fig. 8 each show an embodiment of the present invention, in which 16 is a filter tool made of glass, 17 is a vapor-permeable electrode, 18 is a filter made of a vapor-permeable insulating material, 19 is a terminal, 20 is a vapor-permeable electrode having terminals 19 at both ends, 21 is a closed area, 22 is a level surface, 23 is a center part, and 24 is a tip part.

Fig. 1 shows one embodiment of the present invention, in which the filter 18 such as filter paper or a sieve is sandwiched between one vapor-permeable electrode 17 made of carbon fibers, graphite, gold, platinum, ferrum, titanium, or carbon and the other vapor-permeable electrode 17 made of the same material is inside the filter tool 16. Where an aqueous solution, seawater, blood and other solutions are passed through the inside of the laminate electrode composed of the vapor-permeable electrodes 17 and the filter 18, the microorganisms such as bacteria, viruses and others existing in the solution may be removed therefrom.

In Fig. 1, where electric voltage is applied to the electrodes 17 inside the filter tool 16 and an exhaust gas to be discharged from a flue or exhaust duct is introduced into the inside of the filter tool 16, the carbon dioxide, nitrogen compounds, sulfur compounds and soot contained in the exhaust gas as discharged from the flue or exhaust duct may be efficiently electrically decomposed or recovered.

Fig. 2 shows another embodiment of the present invention, in which the number of the vapor-permeable electrodes 17 and the filters 18 inside the filter tool 16 is increased so as to elevate the filtration efficiency of the device.

Fig. 3 shows yet another embodiment of the present invention, in which nothing is between the two vapor-permeable electrodes 17 provided separately from each other.

Fig. 4 shows an embodiment of the present invention, in which the filter tool 16 has inside thereof a vapor-permeable electrode 20 having terminals 19 at both ends of a filter made of carbon fibers, graphite, gold, platinum, ferrum, titanium, or carbon, such as filter paper or a sieve, and an aqueous solution, seawater, blood and other solutions are passed through the device so as to remove the microorganisms such as bacteria, viruses and others that may exist in the solution.

Since the center part of the vapor-permeable electrode 20 inside the filter tool 16 has a potential difference of 0 (zero), it has a closed area to prevent the solution introduced into the device from passing through the center part.

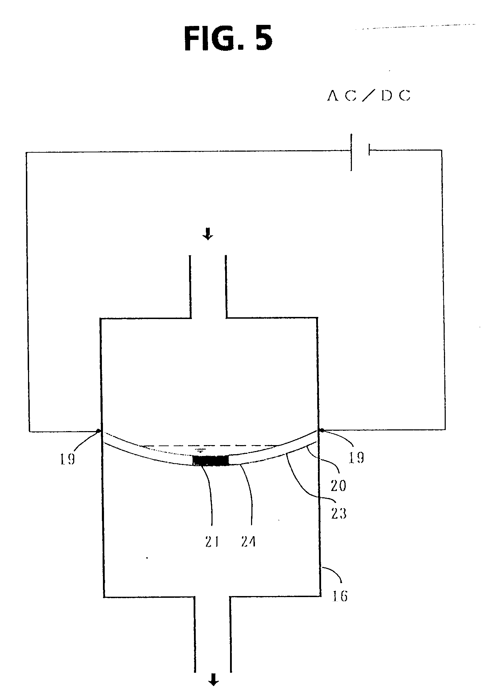

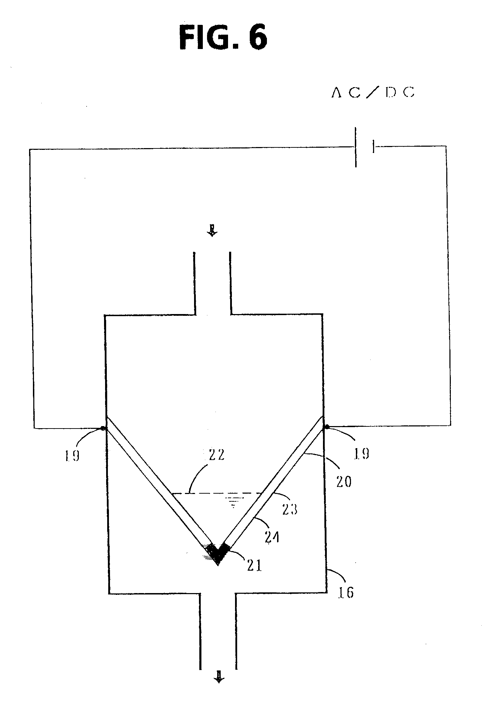

Fig. 5 and Fig. 6 each show another embodiment of the present invention, in which the shape of the vapor-permeable electrode 20 having terminals 19 at the both ends thereof, and which is inside the filter 16, is made concave or coned. As shown in these figures, where the level surface 22 of the liquid is up to near the center part 23 of the vapor-permeable electrode 20 having terminals 19 at both ends thereof, the electric current applied to the device would pass around the outer peripheral part of the vapor-permeable electrode 20 so that only the potential difference remains in the tip part 24 of the concavely curved or conical electrode 20 through which the solution as applied to the device is passing and, as a result, electrolysis of the solution is almost impossible.

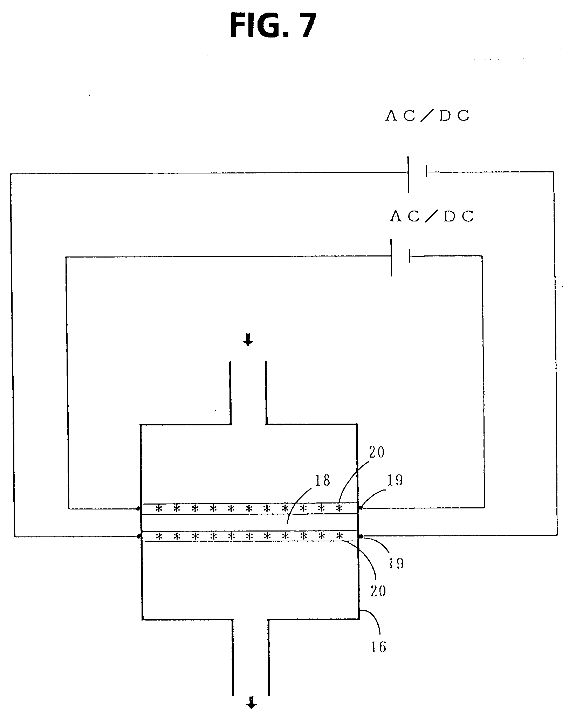

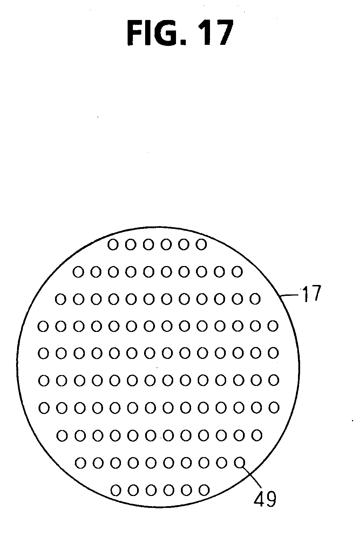

Fig. 7 shows still another embodiment of the present invention, in which a filter 18 made of a vapor-permeable insulating material, such as filter paper or a sieve, is sandwiched between one vapor-permeable electrode 20 having terminals at both ends thereof and the other vapor-permeable electrode 20 of the same type to be a laminate electrode. The filer tool 16 of Fig. 17 thus has a laminate electrode composed of the vapor-permeable electrodes 20 and the filter 18 inside thereof.

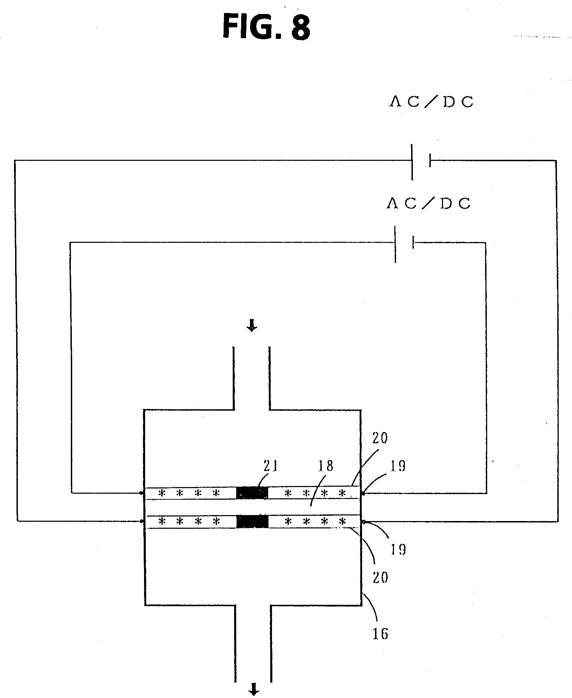

Fig. 8 shows an embodiment of the present invention in which the filter tool 16 has plural vapor-permeable electrodes 20 each having terminals 19 at both ends thereof, the electrodes 20 being laminated in the longitudinal direction.

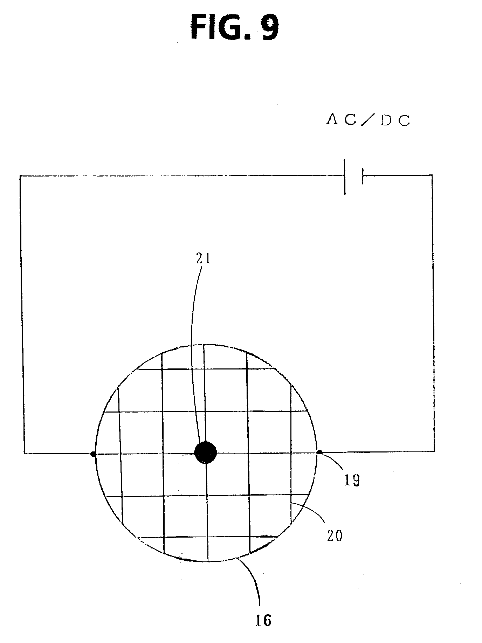

Fig. 9 is a plan view of the vapor-permeable electrode 20 having terminals 19 at both ends thereof, which is inside the filter tool 16.

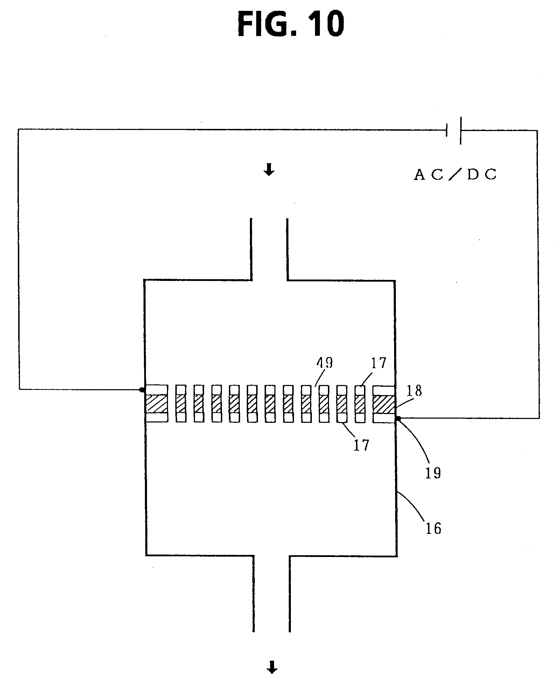

Fig. 10 shows an embodiment of the present invention, in which the filter tool 16 has a laminate of vapor-permeable electrodes 17 and filters 18 inside thereof, the filter 18 being sandwiched between two vapor-permeable electrodes 17. In this type of device, an aqueous solution, seawater, blood and other solutions are passed through the inside of the vapor-permeable electrodes 17 so as to kill and remove the microorganisms existing in the solution. Using this device, the solution applied thereto may be activated. Therefore, this may also be used as a means of removing fur and other items from the solution applied thereto.

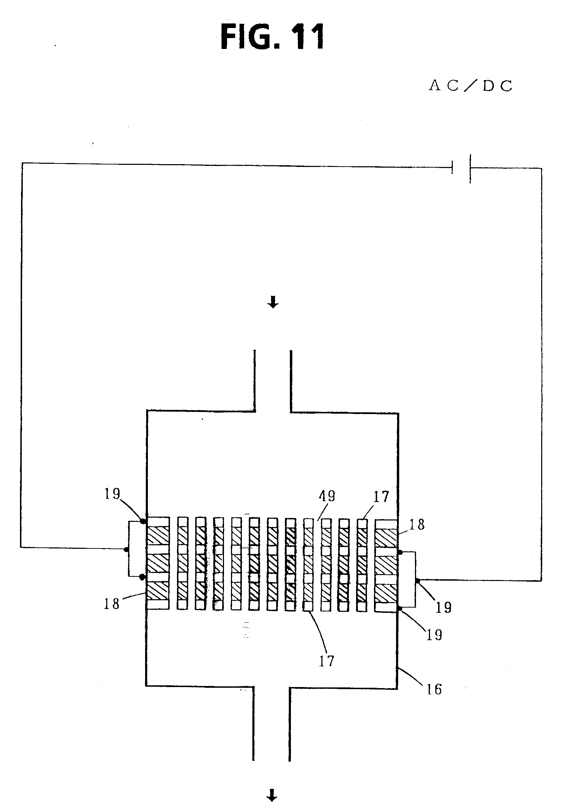

Fig. 11 shows an embodiment of the present invention, in which the numbers of the vapor-permeable electrodes 17 and that of the filters 18 to be laminated inside the filter tool 16 are increased so as to elevate the sterilization efficiency of the device. In the embodiments of Fig. 10 and Fig. 11, since the holes 49 inside the laminate electrode 17 are made linear, even a highly viscous blood, blood component or the like may well pass through the inside of the laminate electrode 17.

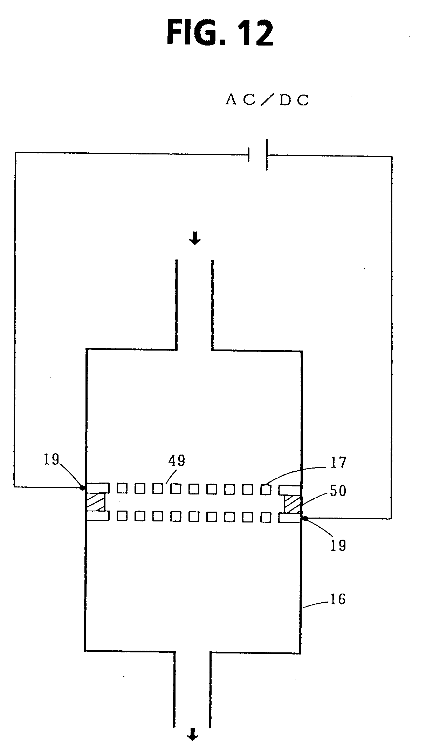

Fig. 12 shows an embodiment of the present invention, in which two flat vapor-permeable electrodes 17 of graphite, carbon, platinum, palladium, nickel, or titanium having holes 49 therethrough have been laminated via packings 50 with a certain distance therebetween. Where an aqueous solution, seawater, blood and other solutions are passed through the inside of the filter tool 16 having such a constitution, microorganisms such as bacteria, viruses and others existing in the solution can be killed.

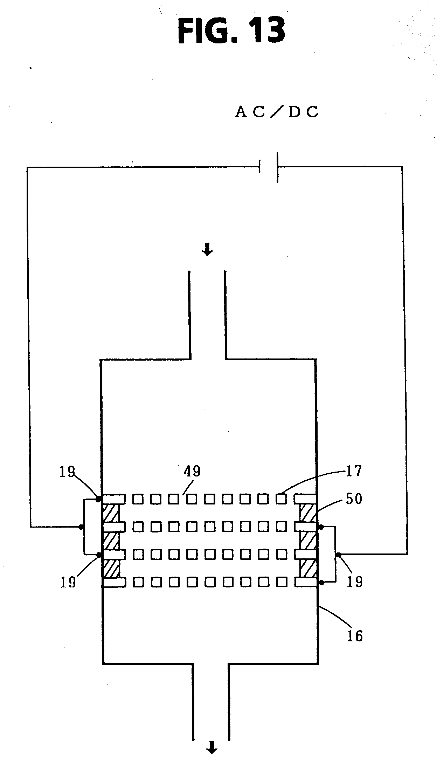

Fig. 13 shows an embodiment of the present invention, which is different from the embodiment of Fig. 12 in that four flat vapor-permeable electrodes 17 of graphite, carbon, platinum, palladium, nickel, or titanium having holes 49 therethrough have each been laminated via packings 50 with a certain distance therebetween. Since this has a greater number of vapor-permeable electrodes being laminated than the embodiment of Fig. 12, the sterilization effect of this may be greatly elevated. As shown in Fig. 13, the more vapor-permeable electrodes 17 that are laminated, the higher the sterilization effect by the device.

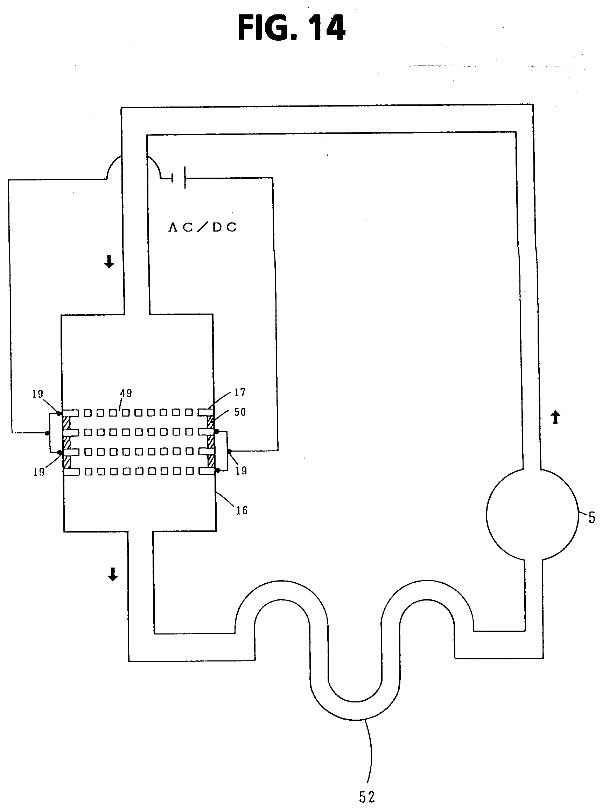

Fig. 14 shows a method of using a filter tool 16 having therein a laminate electrode composed of plural vapor-permeable electrodes 17 laminated via packings 50 therebetween, in which heavy water is circulated inside the filter tool 16 by the use of a pump 5 so as to cause continuous room-temperature nuclear fusion (heat generation by electrolysis), where the generated heat is recovered by the use of a heat exchanger 52.

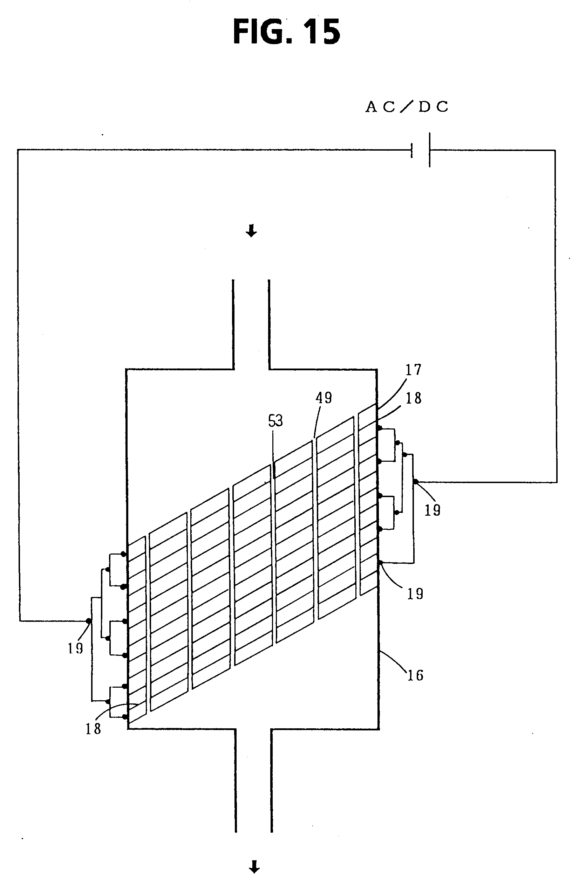

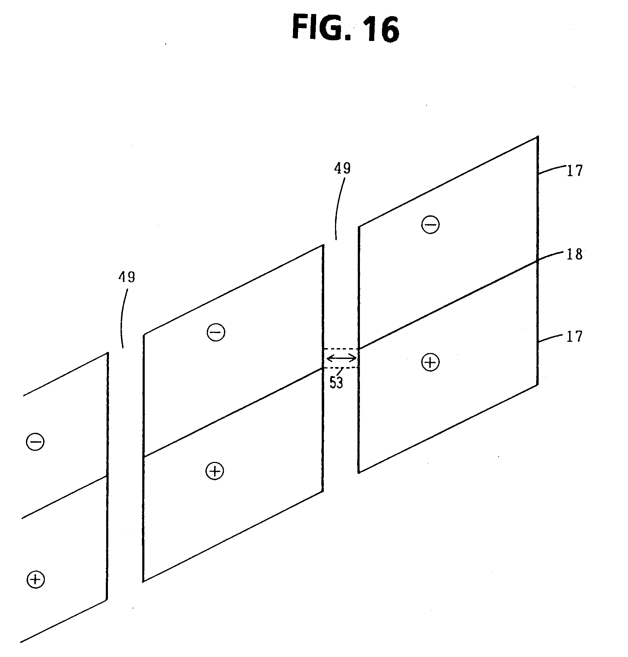

Fig. 15 shows an embodiment of the present invention, in which the electrodes 17 and the insulators 18 are disposed inside the filter tool 16 to have some inclination to the filter tool 16 in such a way that the holes 49 through the inclined electrodes 17 and the insulators 18 are vertical to the filter tool 16. The constitution of the device makes the positive electrode 17 and the negative electrode 17 face each other inside the hole 49 so as to promote the reaction between them. As is shown in Fig. 16, which is an enlarged view of Fig. 15, since the positive electrode 17 and the negative electrode 17 may form an area 53 (as surrounded by the dotted lines in Fig. 16) where the two face each other, the current applied to the system flows from the positive electrode 17 to the negative electrode in the direction of the arrow shown in Fig. 16; and since the current flows across the solution as it flows through the hole 49, the potential gradient may be controlled by adjusting the diameter of the hole 49 so that the electrolytic reaction of room-temperature nuclear fusion (heat generation by electrolysis) of using heavy water or the like may be promoted continuously in the device. In addition, even highly viscous liquids such as blood may smoothly pass through the electrode system, while microorganisms such as bacteria, viruses and others in the blood as applied thereto, and also polluted water and others, may be killed or removed continuously, even when an extremely low electric current is applied to the device.

Where a direct current is applied to the vapor-permeable electrodes 17 as provided inside the filter tool 16, as shown in Fig. 10, Fi. 11, Fig. 12, Fig. 13, Fig. 14 and Fig. 15, it is better that the solution to be treated with the device is passed through the electrodes in the direction from the negative electrode 17 to the positive electrode 17 with the negative electrode 17 being above the positive electrode 17 than that the solution is passed through them in the direction from the positive electrode 17 to the negative electrode 17 with the positive electrode 17 being above the negative electrode 17, since the microorganisms such as bacteria, viruses and others in the solution may be killed more effectively.

Fig. 17 is a plan view of one embodiment of the vapor-permeable electrode 17 for use in the present invention, which is a flat sheet of graphite, carbon, platinum, palladium, nickel, or titanium having holes 49 therethrough.

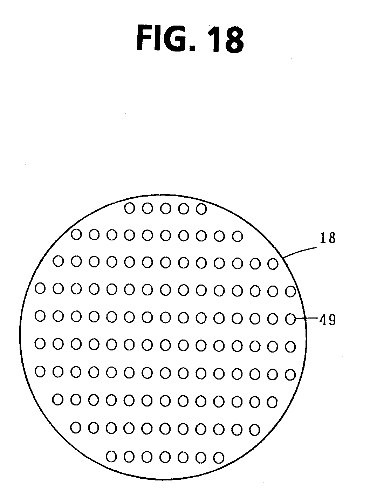

Fig. 18 is a plan view of one embodiment of the filter 18 for use in the present invention, which is a flat sheet of an insulating material of ceramics or Teflon (trade name by E. I. DuPont de Nemours & Co.) having holes 49 therethrough.

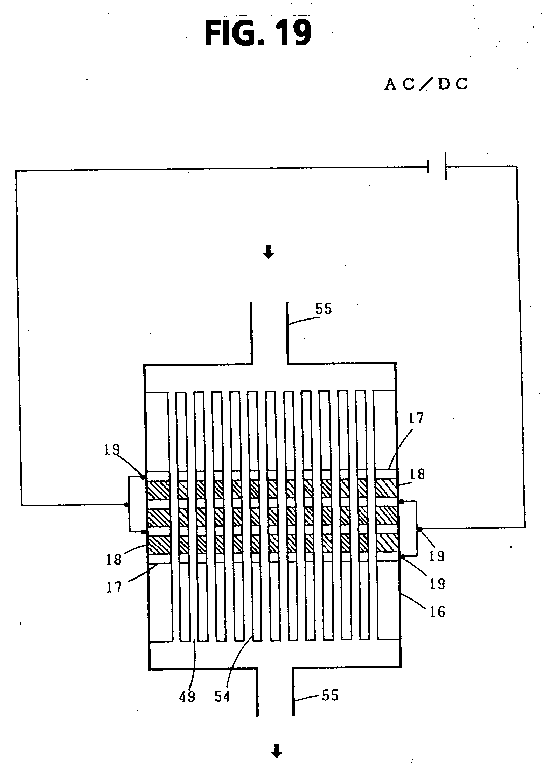

Fig. 19 shows another embodiment of the present invention, in which plural electrodes 17 and plural insulators 18 have been laminated to form a laminate electrode 17 having holes 49 therethrough inside the filter tool 16. If city tap water, sewage, blood or other solutions are directly passed through the holes 49, substances which are incompatible with human bodies would often be dissolved out from the electrodes and others or blood would often coagulate. In order to avoid these problems, tubes 54 made of a ferroelectric chemical product with a high dielectric constant or other substances which are compatible with human bodies, such as Teflon, polyolefins, polyesters, polyethylene or barium titanate ceramics, are inserted inside the respective through-holes 49 of the laminate electrode 17. The plural tubes 54 inserted inside the through-holes 49 of the laminate electrode 17 are connected to the connecting pipes 55 with the inside of the connected tubes being in an electric field, and city tap water, sewage, blood or other solutions are passed through the holes 49 so as to kill the microorganisms such as viruses, bacteria and others in the solution or to remove the polluting substances.

Where blood is passed through the inside of the electrodes 17 and the insulators 18 in the system of Fig. 19, the tubes 54 which are compatible with human bodies must previously be inserted inside the respective holes 49 so as to coat the surfaces of the electrodes 17 and the insulators 18 with the tubes 54, or a resin which is compatible with human bodies, such as Heparin (trade name), must previously be coated on the surfaces of the electrodes 17 and the insulators 18 in order that the coated electrodes and insulators may be made compatible with human bodies. If not, the blood applied to the device would coagulate.

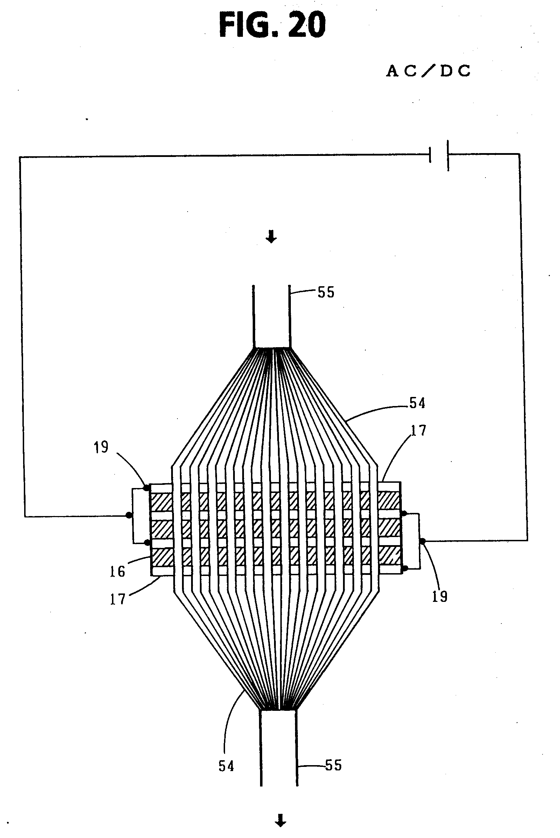

Fig. 20 shows an embodiment of the present invention, which is different from the embodiment of Fig. 19 in that the plural tubes 54 have been bundled into one with an adhesive to be connected to the connecting pipe 55.

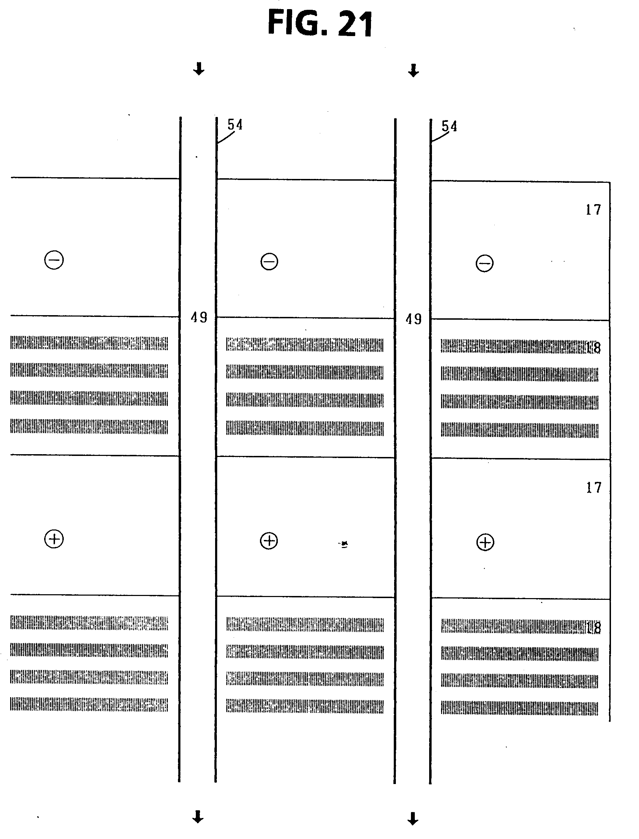

Fig. 21 is an enlarged view of the laminate electrode 17 as shown in Fig. 19 and Fig. 20, in which plural electrodes 17 and plural insulators 18 have been laminated.

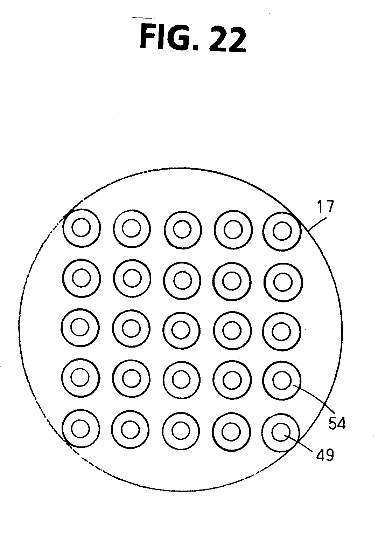

Fig. 22 is a plan view of one embodiment of the vapor-permeable electrode 17 for use in the present invention, which is a flat sheet of graphite, carbon, platinum, palladium, nickel, or titanium having holes 49 therethrough. In this case, tubes 54 made of a body-compatible chemical product have been inserted inside the holes 49.

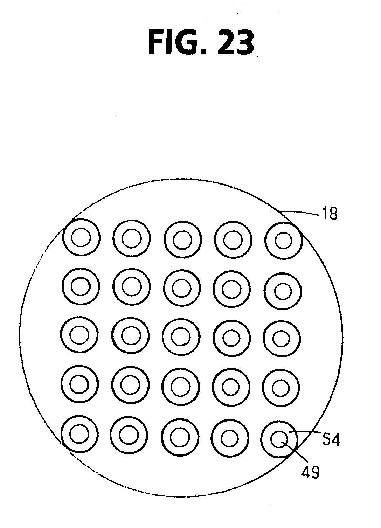

Fig. 23 is a plan view of one embodiment of the filter or insulator 18 for use in the present invention, which is a flat sheet of an insulating material such as ceramics or Teflon having holes 49 therethrough. In this case, tubes 54 made of a body-compatible chemical product have been inserted inside the holes 49.

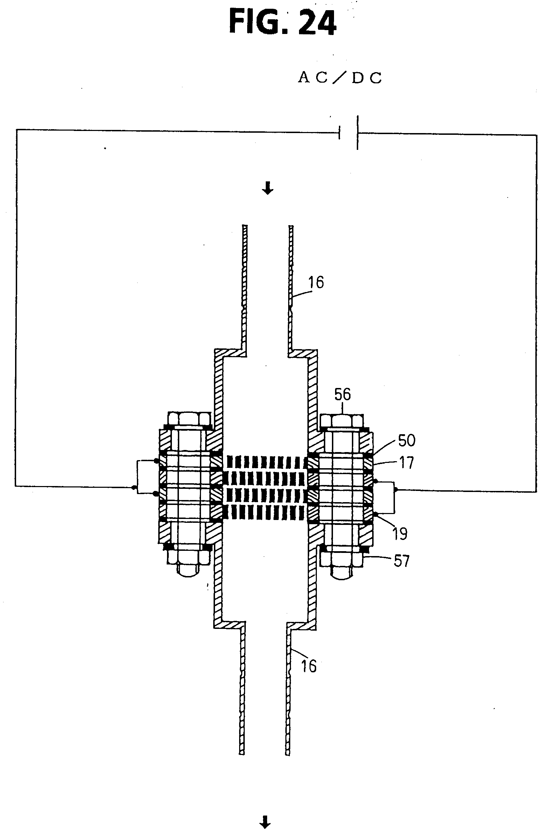

Fig. 24 is an outline view of an embodiment of the present invention, which is different from the outline views of Fig. 10, Fig. 11, Fig. 12, Fig. 13 and Fig. 14. This shows a practical model of the four-layered laminate electrode 17 mentioned above. As a result of the experiment using the model where a voltage from several volts to about 10 volts was applied between the electrodes, it was verified that the influence by the generated electric field displayed the theoretically calculated microbicidal effect in killing the microorganisms such as viruses, bacteria and others in the solution applied to the device.

The embodiment of Fig. 24 is such that plural negative electrodes 17 and positive electrodes 17 have been alternately laminated with a space therebetween inside the filter tool 16, the electrodes 17 being flat sheets made of graphite, gold, platinum, ferrum, titanium, palladium, or carbon and having small holes therethrough. Where an exhaust gas as discharged from a flue or exhaust duct is passed through the inside of the filter tool 16, the carbon dioxide, nitrogen compounds, sulfur compounds, soot and others in the exhaust gas as discharged from the flue or exhaust duct may be efficiently electrically decomposed or recovered. In order to more effectively recover the soot, though not shown in Fig. 24, a heating element such as nichrome wires should be provided between the electrodes 17 made of graphite so as to heat the inside of the filter tool 16 up to a temperature falling within the range of approximately 600°C to 700°C, or a direct current or alternating current is additionally applied to the electrodes 17 made of graphite conductive material separately from the current originally applied to the electrodes 17 so as to heat the electrodes 17 made of graphite up to a temperature falling within the range of approximately 600°C to 700°C, whereby the soot electrically absorbed onto the surfaces of the electrodes 17 is fired on while it is still on the surfaces of the electrodes 17.

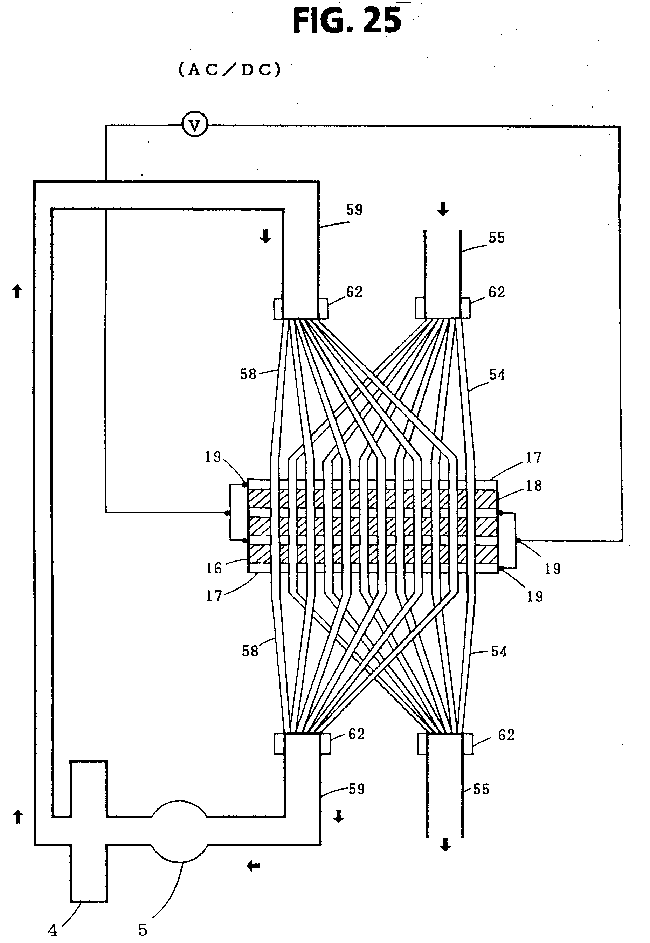

Fig. 25 shows another embodiment of the present invention. In the illustrated device, where an alternating current or a high frequency current is applied to the laminate electrode 17 composed of plural vapor-permeable electrodes 17 and plural filters 18, the current flows therethrough, while a direct current is not being applied thereto; and when an alternating current or high frequency current higher than a determined current value is applied to the device, then the laminate electrode generates Joule's heat. In order to lower the thus heated laminate electrode to a determined temperature or lower, plural tubes 58 for circulation of cooling water therethrough are inserted and attached to the inside of the holes 49 as formed through the laminate electrode 17 and they are bundled into one to be connected to a cooling water-circulating pipe 59. An aqueous solution cooled in a cooling device 4 is circulated inside the cooling water-circulating tubes 58 by the use of a pump 5 so as to indirectly cool the laminate electrode 17. Accordingly, when blood is passed through the inside of the tubes 54 as inserted into the laminate electrode 17 for sterilization of it, proteins and other substances in the blood which are not resistant to heat are not denatured or deteriorated.

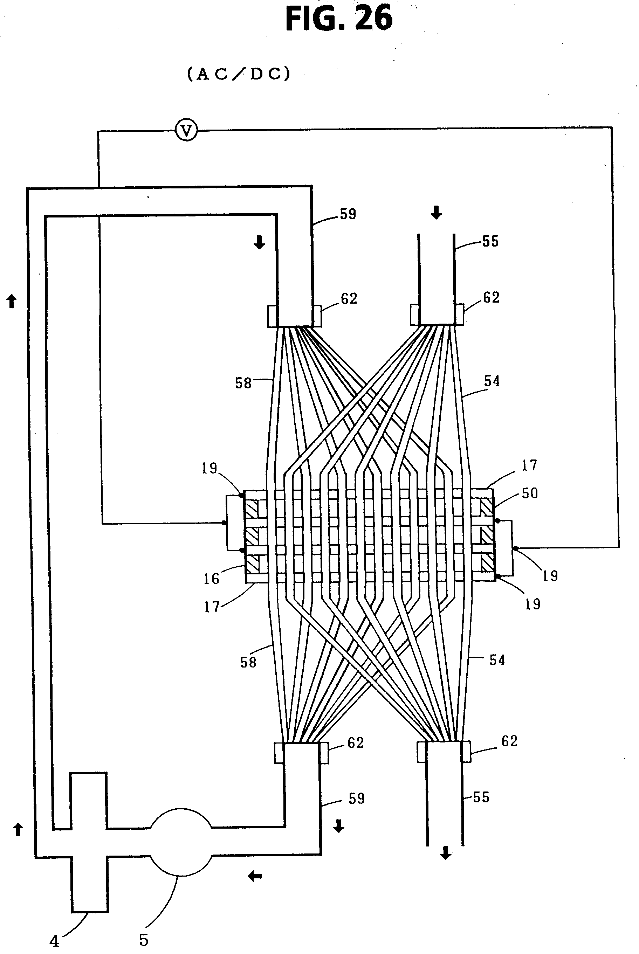

Fig. 26 shows an embodiment of the present invention, which is different from the embodiment of Fig. 25 in that the laminate electrode 17 of the latter has the filters 18 while the laminate electrode 17 of the former has packings 50 made of an insulating material in place of the filters 18.

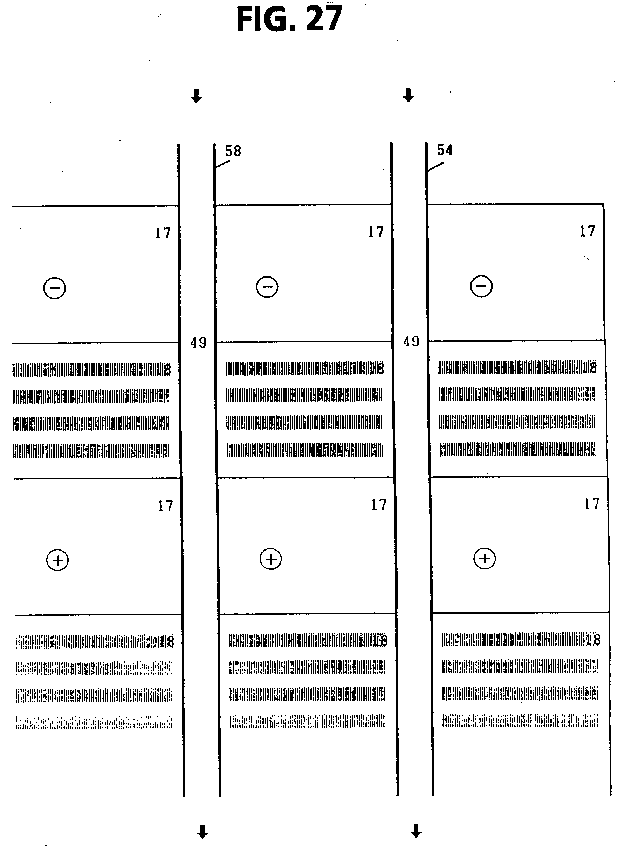

Fig. 27 is an enlarged longitudinal sectional view of the laminate electrode 17 of Fig. 25, which is composed of the vapor-permeable electrodes 17 and the filters 18.

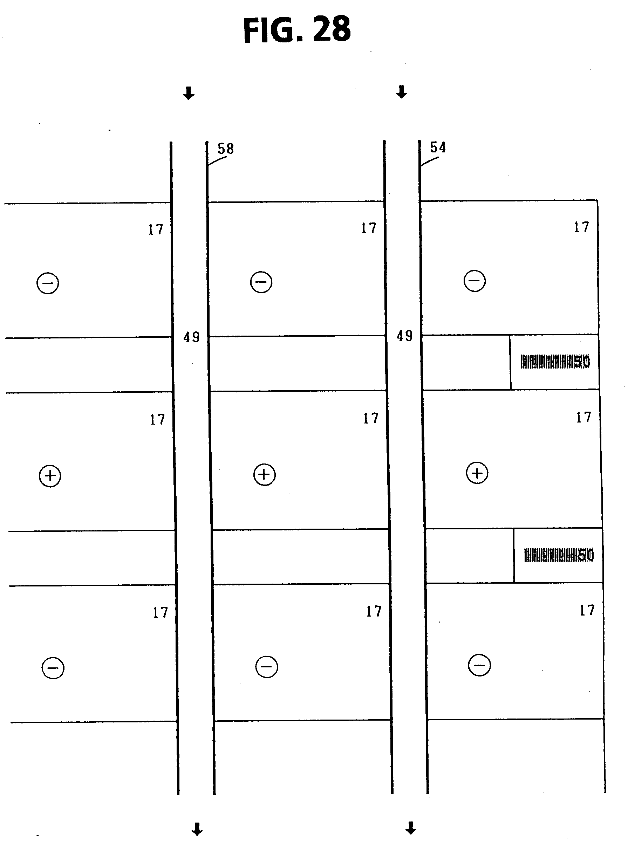

Fig. 28 is an enlarged longitudinal sectional view of the laminate electrode 17 of Fig. 26, which is composed of the vapor-permeable electrodes 17 and the insulating packings 50.

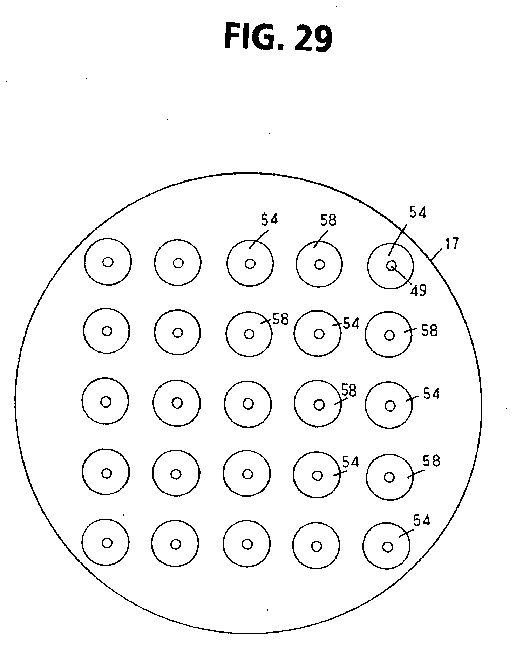

Fig. 29 is a plan view of the vapor-permeable electrode 17 of Fig. 25 and Fig. 26.

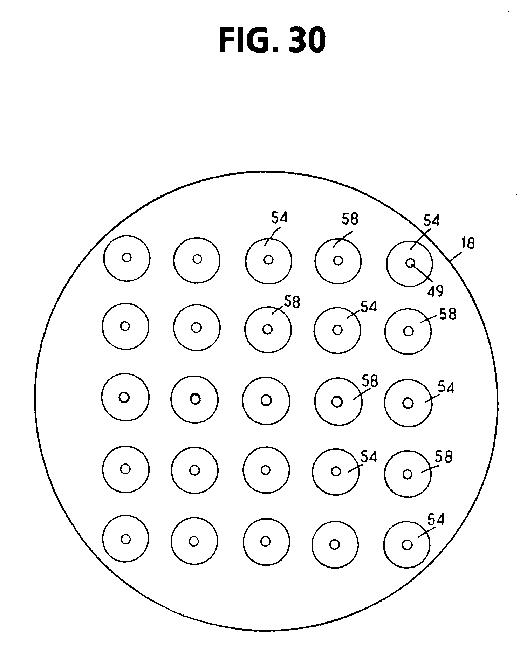

Fig. 30 is a plan view of the filter 18 shown in Fig. 25.

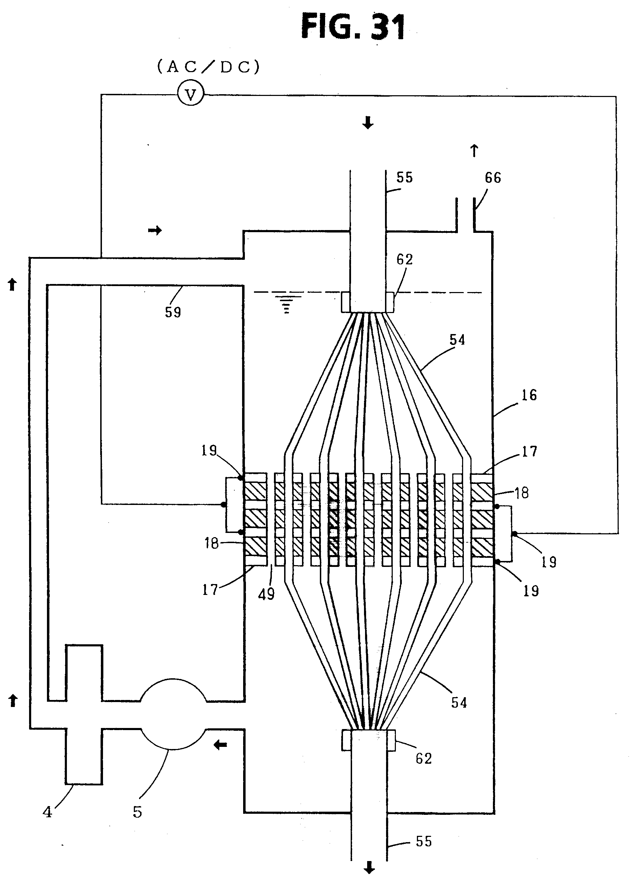

Fig. 31 shows another embodiment of the laminate electrode 17 for use in the present invention, which is composed of the vapor-permeable electrodes 17 and the filters 18 and which is provided with a cooling means for directly cooling the laminate electrode 17. Specifically, an aqueous solution cooled in the cooling device 4 is fed and circulated inside the filter tool 16 by the use of the pump 5 so as to cool the inside of the filter tool 16. The cooled aqueous solution directly passes through the holes 49 of the vapor-permeable electrodes 17 and the filters 18 made of a vapor-permeable insulating material whereupon the cooled aqueous solution is kept in contact with the laminate electrode 17 to directly remove the Joule's heat as generated by the laminate electrode 17.

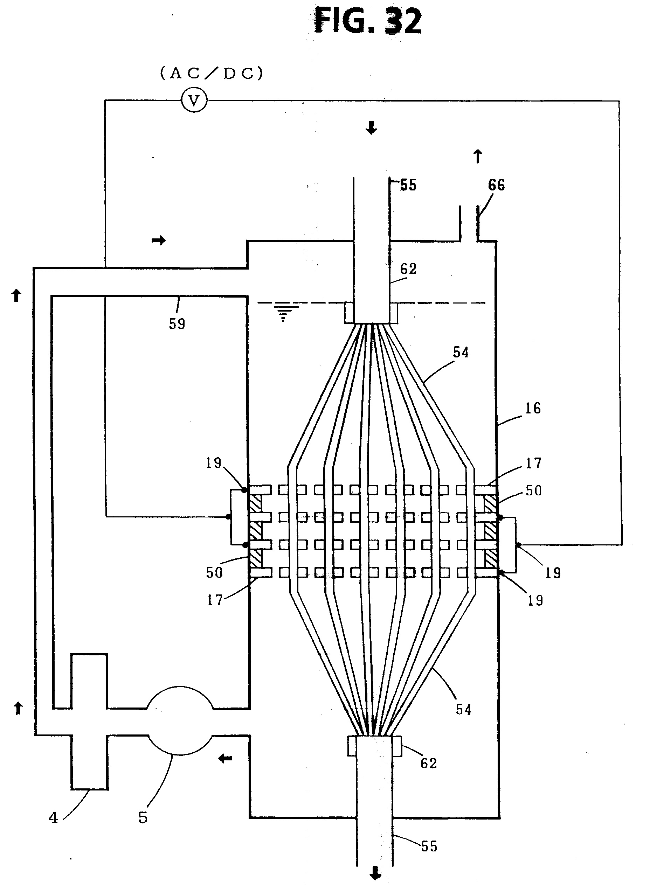

Fig. 32 shows still another embodiment of the present invention, which is different from the embodiment of Fig. 31 in that the laminate electrode 17 of the latter has the filters 18 while the laminate electrode 17 of the former has the packings 50 made of an insulating material.

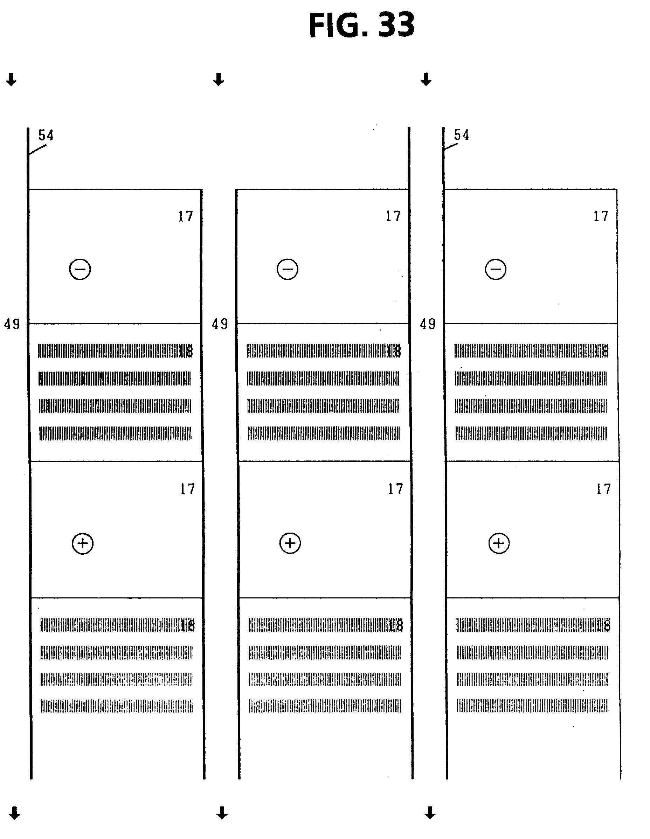

Fig. 33 is an enlarged longitudinal sectional view of the laminate electrode 17 of Fig. 31, which is composed of the vapor-permeable electrodes 17 and the filters 18.

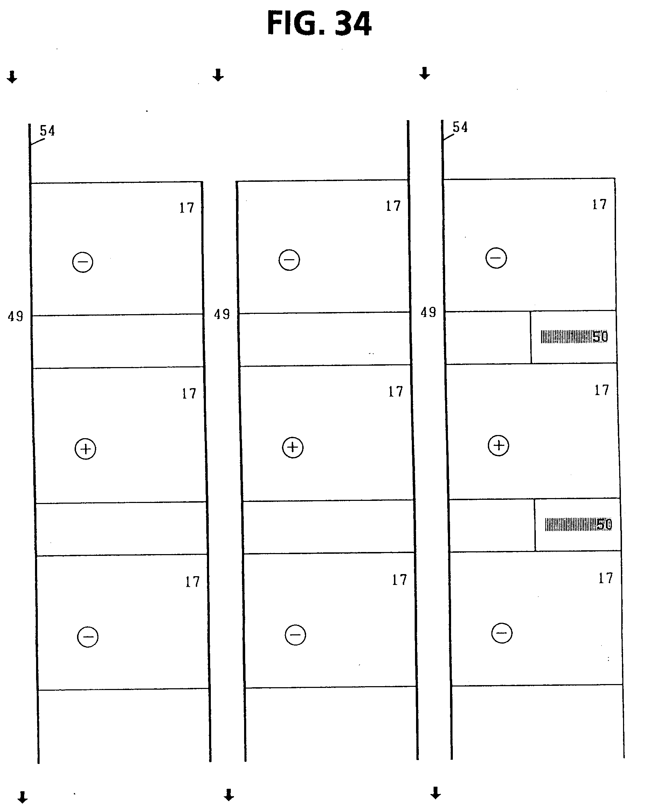

Fig. 34 is an enlarged longitudinal sectional view of showing the laminate electrode 17 of Fig. 32, which is composed of the vapor-permeable electrodes 17 and the packings 50 made of an insulating material.

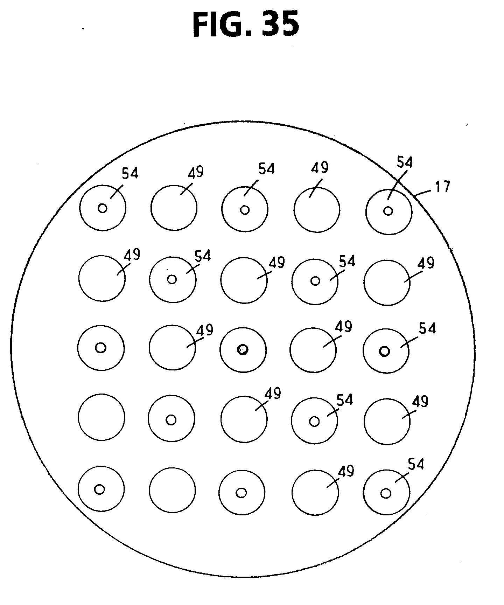

Fig. 35 is a plan view of showing the vapor-permeable electrode of Fig. 31 and Fig. 32.

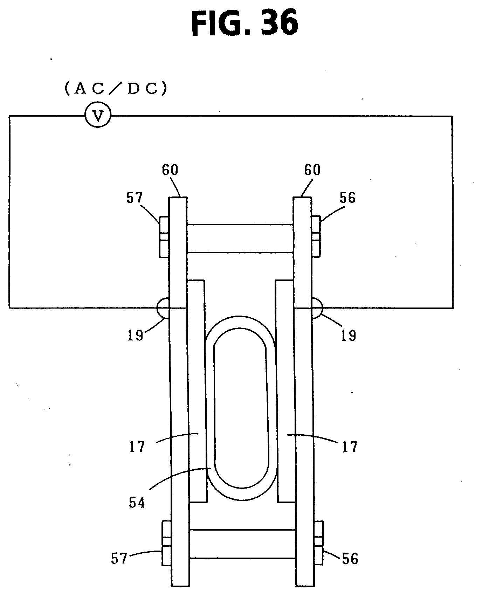

Fig. 36 shows still another embodiment of the present invention, in which the tube 54 made of a ferroelectric material having a high dielectric constant, such as Teflon, polyolefins, polyesters, polyethylene or barium titanate ceramics, has been sandwiched between the electrode 17 and the electrode 17. In the illustrated case, an electric voltage is directly applied to the tube 54 to generate an electric field inside the tube 54, whereby microorganisms such as bacteria, viruses and others in the city tap water, sewage, blood and other solutions passing through the inside of the tube 54 are killed. Also in the case of Fig. 36, when an alternating voltage or a high frequency voltage is applied to the electrodes 17 and when the current therethrough becomes more than a determined one, then the electrodes 17 generate Joule's heat, as in the case of Fig. 25. Therefore, it is necessary to cool the electrodes 17 so that the substance passing through the inside of the tube 54 may be cooled to a temperature at which the proteins and others in the substance are not denatured or deteriorated.

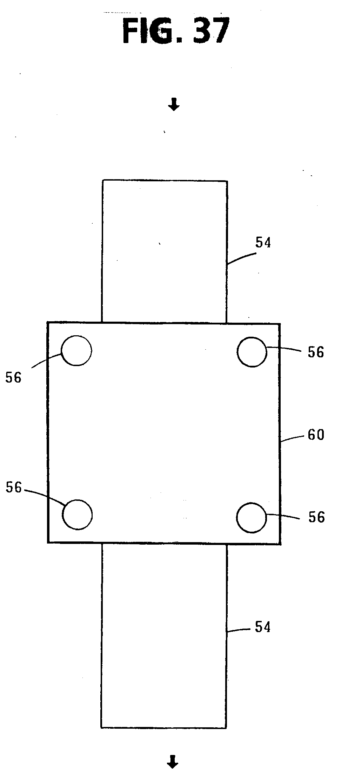

Fig. 37 is a side view of the outline view of Fig. 36, which indicates the direction of the city tap water, sewage, blood or other solutions to pass through the inside of the tube 54.

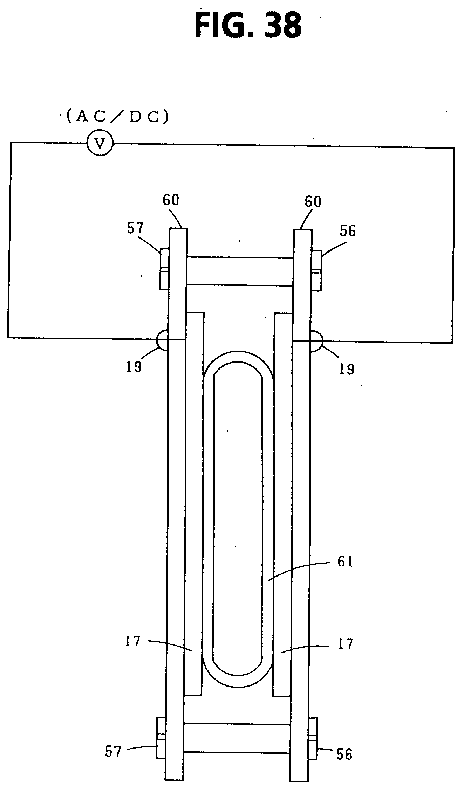

Fig. 38 shows still another embodiment of the present invention, in which collected blood has been put in a blood collecting bag 61 made of a ferroelectric substance having a high dielectric constant, such as Teflon, polyolefins, polyesters, polyethylene or barium titanate ceramics, and the blood-containing bag 61 has been sandwiched between the electrodes 17 and 17. In this case, an electric voltage is applied to the blood-containing bag 61 to generate an electric field inside the bag 61 whereby the microorganisms such as bacteria, viruses and others existing in the blood in the bag 61 are killed. Also, in the case of Fig. 38, when an alternating voltage or a high frequency voltage is applied to the electrodes 17 and when the current therethrough becomes more than a determined one, then the electrodes 17 generate Joule's heat, as in the case of Fig. 36. Therefore, it is necessary to cool the electrodes 17.

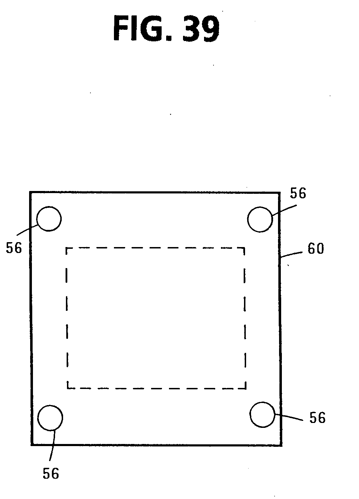

Fig. 39 is a side view of the outline view of Fig. 38.

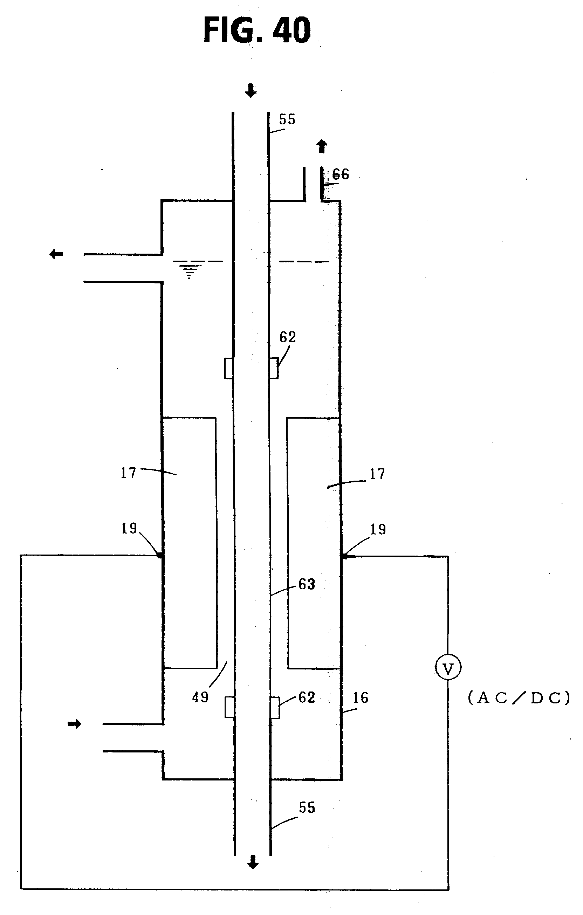

Fig. 40 shows an embodiment of the present invention, in which one electrode 17 faces another electrode 17 inside the filter tool 16 and a pipe 63 made of a semi-permeable membrane has been sandwiched between the two facing electrodes 17. An aqueous solution or an aqueous saline solution having a salt concentration near that of a physiological saline solution or an aqueous solution of other components is passed and circulated through the inside of the filter tool 16 in the direction of the arrows shown in Fig. 40, whereupon an electric voltage is applied to the electrodes 17 in the filter tool 16 so that an electric current is applied inside the filter tool 16. Although only one semi-permeable pipe 63 is shown in Fig. 40, about 10,000 semi-permeable pipes 63 are provided in the actual plant. City tap water, sewage, blood, seawater or other solutions are passed through the inside of the filter tool having the semi-permeable pipe 63 while applying an electric current to said aqueous solution or aqueous saline solution, which is kept in direct contact with the electrodes 17, whereupon the applied current passes through the semi-permeable pipe 63 so that an indirect current is applied to the city tap water, sewage, blood, seawater or other solutions passing through the inside of the semi-permeable pipe 63. Accordingly, due to the microbicidal effect of the current, microorganisms such as bacteria, viruses and others existing in said solutions can be killed. The filter tool 16 of Fig. 40 may be considered to be a conventional filter tool for hemodialysis as provided with the electrodes 17.

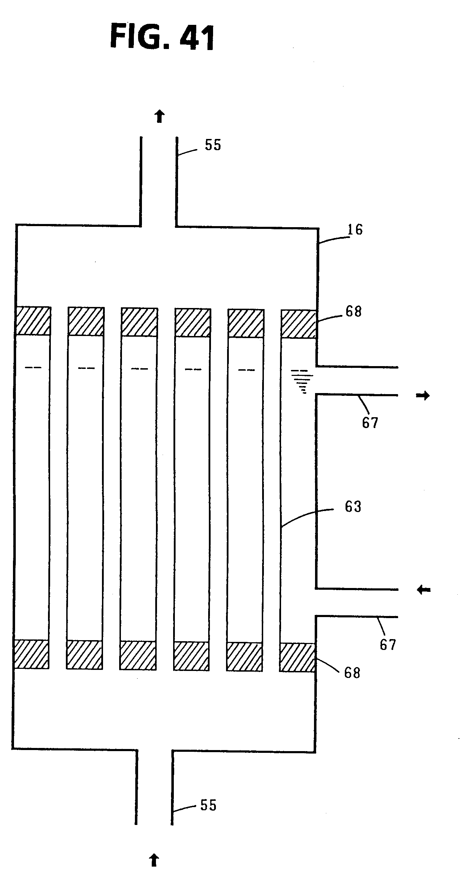

Fig. 41 is an outline view of a conventional artificial kidney machine for hemodialysis.

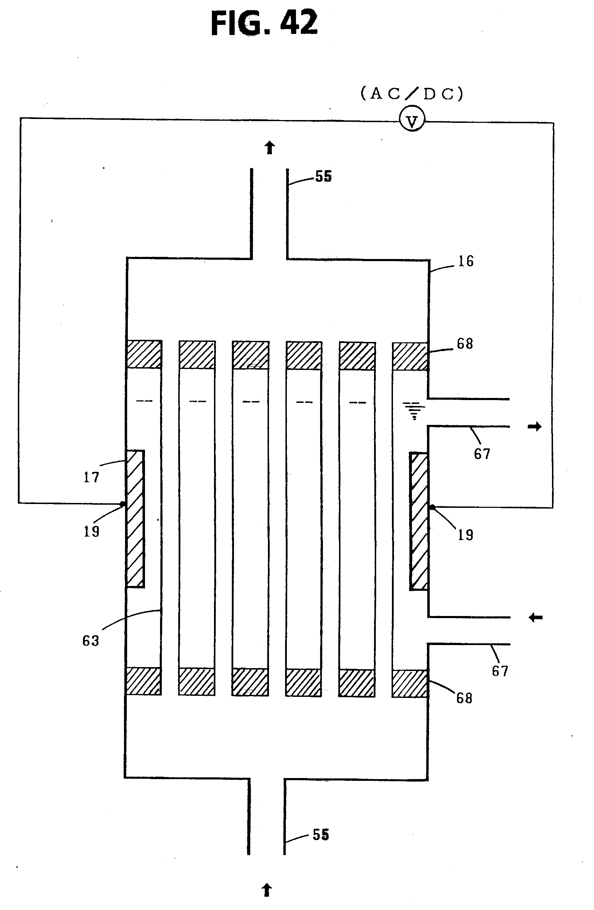

Fig. 42 is a modification of the system of Fig. 41, in which the conventional artificial kidney machine for hemodialysis is provided with electrodes 17. In the embodiment of Fig. 42, a dialysate is fed into the inside of the filter tool 16 in the direction of the drawn arrow from the connecting pipe 67, while city tap water, sewage, blood, seawater or other solutions are passed through the inside of the semi-permeable pipe 63 in the direction of the illustrated arrow, and while applying electric voltage to the electrodes 17 to send an electric current through the dialysate, whereby an electric current is indirectly sent through the city tap water, sewage, blood, seawater or other solutions passing through the inside of the semi-permeable pipe, via the dialysate. Accordingly, microorganisms such as viruses, bacteria and others existing in the city tap water, sewage, blood, seawater or other solutions as passing through the inside of the semi-permeable pipe can be killed.

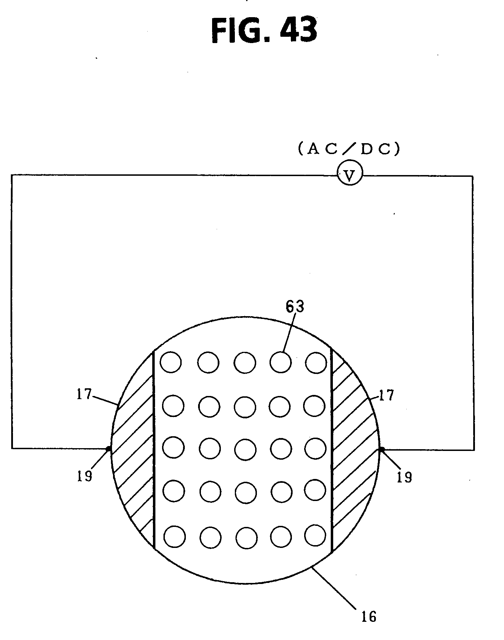

Fig. 43 is a lateral sectional view of Fig. 42.

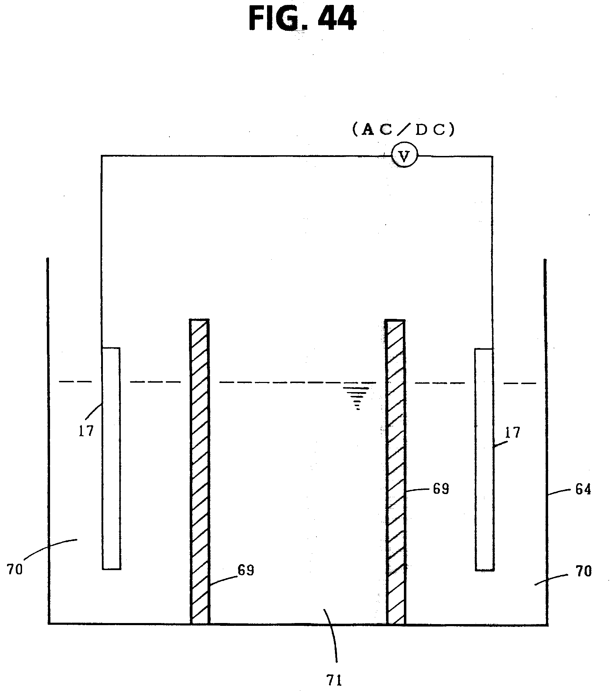

Fig. 44 shows an embodiment of the present invention, in which the inside of the container 64 made of an insulating material which has been divided by two semi-permeable membranes 69 and the electrodes 17 have been put in the outside sections separated by the two semi-permeable membranes 69. City tap water, sewage, blood, seawater or other solutions are put inside the container 64 made of an insulating material and an electric voltage is applied to the electrodes 17 so as to apply an electric current to the city tap water, sewage, blood, seawater or other solutions, thereby sterilizing them.

Where blood needs to be sterilized by the system of Fig. 44, the solution inside the divided areas 70 of the container 64 made of an insulating material must be a physiological saline solution. Since none of the chloride ion and other ions generated by the electrolysis penetrate or move into the blood in the illustrated system, blood may safely be sterilized in the system by applying an electric current thereto.

While the invention has been described in detail with reference to specific embodiments thereof, it will be apparent to one skilled in the art that various changes and modifications can be made therein without departing from the spirit and scope thereof.

The features disclosed in the foregoing description, in the claims and/or in the accompanying drawings may, both separately and in any combination thereof, be material for realising the invention in diverse forms thereof.

## Claims

1. A filtration method using a laminate electrode composed of electrode(s) made of a vapor-permeable conductive material and filter(s) made of a vapor-permeable insulating material, in which an exhaust gas as discharged from some equipment is passed through the inside of the laminated vapor-permeable electrode(s) and filter(s) so as to decompose or recover the carbon dioxide, nitrogen compounds, soot and the like contained in the exhaust gas.

2. A filtration method using a filter device having two or more electrodes made of a vapor-permeable conductive material, in which an exhaust gas discharged from some equipment is passed through the inside of the filter device so as to decompose or recover the carbon dioxide, nitrogen compounds, or soot contained in the exhaust gas.

3. A filtration method using a filter device having two or more electrodes made of a vapor-permeable conductive material, in which an additional direct current or alternating current is applied to the electrodes separately from the current originally applied to them so as to heat the electrodes up to a temperature of approximately 600°C to 700°C, and an exhaust gas as discharged from some equipment is passed through the inside of the filter device having the heated electrodes so as to decompose or recover the carbon dioxide, nitrogen compounds, or soot contained in the exhaust gas.

4. A filtration method using a laminate electrode composed of electrode(s) made of a vapor-permeable conductive material and filter(s) made of a vapor-permeable insulating material, in which a solution or vapor is passed through the inside of the laminated vapor-permeable electrode(s) and filter(s) so as to remove microorganisms such as bacteria, viruses and others existing in the solution or vapor by killing them or absorbing them into the laminate electrode.

5. A filtration method using a filter device having two or more electrodes made of a vapor-permeable conductive material, in which city tap water, sewage, seawater, blood and other solutions are passed through the inside of the filter device so as to kill microorganisms such as bacteria, viruses and others existing in the solutions.

6. The filtration method as claimed in claim 4, in which, when a direct current is applied to the vapor-permeable electrode(s) provided inside the filter device, the solution to be treated is passed through the inside of the filter device in a direction from the negative electrode to the positive electrode.

7. A filtration method using vapor-permeable electrode(s) each having terminals at both ends, in which city tap water, sewage, seawater, blood and other solutions are applied to the electrode(s) so as to kill and remove microorganisms such as bacteria, viruses and others existing in the solutions.

8. A filtration method using electrode(s) provided at both ends of a semi-permeable pipe, in which a solution is put in the semi-permeable pipe under indirect electric current as applied thereto and city tap water, sewage, seawater, blood and other solutions are passed through the inside of the semi-permeable pipe so as to kill and remove microorganisms such as bacteria, viruses and others existing in the solutions.

9. A filtration method using a flat container such as a blood collecting bag or the like made of a ferroelectric material having a high dielectric constant, in which electrode(s) is/are provided outside of the flat container, and whereby blood, city tap water, sewage or other solutions are put in the flat container, and an electric voltage is applied to the electrode(s) whereby microorganisms such as bacteria, viruses and others existing in the solutions are removed by killing or absorbing them due to the electric field to be generated by the applied voltage.

10. A filtration method using tube(s) made of a ferroelectric material having a high dielectric constant, in which electrode(s) is/are provided outside of the tube, city tap water, sewage, seawater, blood or other solutions are passed through the inside of the tube(s), and an electric voltage is applied to the electrode(s) whereby microorganisms such as bacteria, viruses and others existing in the solutions as passing through the inside of the tube(s) are removed by killing or absorbing them due to the electric field to be generated by the applied voltage.

11. A filtration method using an electric current of a bi-polar pulse or direct current-multiplied sine curve alternating current or direct current or other electric currents having properties of other alternating currents and direct current, in which microorganisms such as bacteria, viruses and others are killed by the current.

12. A filter device having a laminate of plural electrodes made of a vapor-permeable conductive material, in which the electrodes have been laminated with a space therebetween and housed in an insulating pipe.

13. A filter device having a laminate of electrodes made of a vapor-permeable conductive material and a filters made of a vapor-permeable insulating material, in which the electrodes and the filters have been laminated alternately and housed in an insulating pipe.
